# EUROPÄISCHE PATENTSCHRIFT

(11) **EP 1 443 101 B1**
(45) Veröffentlichungstag und Bekanntmachung des Hinweises auf die Patenterteilung: **22.02.2012**
(21) Anmeldenummer: 03028660.3
(22) Anmeldetag: 16.12.2003
(51) Int. Cl.: B01L 1/00, C12M 1/00

(54) **Klimaschrank mit beweglichem Träger**
Incubator with moveable support
Incubateur avec support mobile

(30) Priorität: 18.12.2002 CH 21722002
(43) Veröffentlichungstag der Anmeldung: 04.08.2004
(73) Patentinhaber: LICONIC AG, 9493 Mauren (LI)
(72) Erfinder: Malin, Cosmas, G., 9493 Mauren (LI)
(74) Vertreter: Sutter, Kurt

(56) Entgegenhaltungen:
- EP-A- 0 569 214
- EP-A- 1 074 488
- EP-A- 1 354 623
- US-A- 5 470 744
- US-A- 5 735 587
- US-B1- 6 475 776

## Beschreibung

Die Erfindung betrifft einen Klimaschrank nach dem Oberbegriff des Patentanspruchs 1.

Automatisierte Klimaschränke eingangs erwähnter Art weisen einen Klimanutzraum und einen Klimagenerator zur Erzeugung kontrollierter klimatischer Bedingungen im Nutzraum auf. Sie werden überall dort benötigt, wo im Nutzraum unter kontrollierten Klimabedingungen gelagerte Objekte automatisch zugreifbar sein müssen. Das Anwendungsgebiet reicht von der langfristigen gekühlten Lagerung von Mikrotiterplatten bis hin zum Bebrüten von Zellkulturen oder Mikroorganismen unter eng tolerierten Klimabedingungen und unter äusserst hohen Reinheitsbedingungen. In Anwendungen, wo Partikel oder Mikroorganismen in einer Lösung schwebend gelagert sein müssen, ist oftmals zusätzlich eine kontinuierlicher Agitation des Lagergutes notwendig.

In der CH 690 645 und der EP 1 074 488 ist eine Einrichtung vorgestellt die eine Benutzertüre und eine automatisch betätigbare Hilfstüre aufweist und im Innern Lagerschächte und ein Liftsystem vorgesehen ist, das zum Transport des Lagergutes zwischen den Lagerschächten und der Hilfstüre dient. Diese Anordnung hat zum Nachteil, dass das Handling, d.h. das Lagersystem und die Vorrichtung zum automatisierten Zu- und Wegführen von Objekten, die Zugänglichkeit zu Boden und Wandungen der Innenseite der Klimakammer erschwert und gar verunmöglicht. Für die Reinigung muss das Handling aus der Klimakammer entfernt werden.

Die in der deutschen Patentanmeldung DE 100 24 581 dargestellte Anordnung weist einen schrägen Boden auf und ein nach oben versetztes Handling auf. Die Zugänglichkeit bei dieser Anordnung ist jedoch nicht ideal. Seiten- und Rückwand bleiben auch hier schlecht oder gar nicht zugänglich. Die Raumnutzung ist durch das Höhersetzen des Handlings ebenfalls nicht optimal. Es ist daher Aufgabe der vorliegenden Erfindung eine Einrichtung eingangs erwähnter Art zu schaffen, die einfacher zu reinigen ist. Es ist auch eine Vorrichtung bereitzustellen, die in einfacher Weise eine Agitation des Lagerguts erlaubt.

Diese Aufgabe wird vom Klimaschrank nach Anspruch 1 gelöst.

Erfindungsgemäss ist die drehfest in der Klimakammer angoerdnete Trägerplatte, auf der der die Lagerschächte angeordnet wird, also beweglich gegenüber dem Nutzaum ausgestaltet, so dass diese beim Reinigen so bewegt werden kann, dass der zu reinigende Bereich besser zugänglich wird, und/oder so dass sie zur Agitation des Lagerguts bewegt werden kann. Weiter ist ein Antrieb zum Rütteln der Trägerplatte vorgesehen.

Die Vorteile bevorzugter Ausführungen der Erfindung sind unter anderem
- die Erweiterung des Anwendungsbereichs automatisierter Inkubatoren durch Agitation
- die effiziente und homogene Agitation, durch Vortexbewegung
- die ungehinderte Zugänglichkeit zu den Innenflächen der Klimakammer durch beweglich angeordnetes Handling
- die Vereinfachung von Wartung und Justage des Handlings durch einfache Entnahme des Handlings

Im folgenden ist die Erfindung in den **Fig. 1-3** erläutert.
- **Fig. 1a**: zeigt die Seitenansicht eines ersten Ausführungsbei- spiels eines erfindungsgemässen Klimaschrankes mit ver- schiebbarem Lagersystem.
- **Fig. 1b**: zeigt die Frontansicht des ersten Ausführungsbeispiels des erfindungsgemässen Klimaschrankes mit verschiebba- rem Lagersystem.
- **Fig. 2a**: zeigt die Frontansicht eines zweiten Ausführungsbeispiels eines erfindungsgemässen Klimaschrankes mit einem teilweise schwenkbaren Lagersystem.
- **Fig. 2b**: zeigt die Frontansicht eines zweiten Ausführungsbeispiels eines erfindungsgemässen Klimaschrankes mit einem teilweise kippbaren Lagersystem.
- **Fig. 3**: zeigt die Gesamtansicht eines Ausführungsbeispiels eines erfindungsgemässen Klimaschrankes mit einem teilweise verschiebbaren Lagersystem.
- **Fig. 4**: zeigt eine Ansicht von Trägerplatte und Grundplatte eines Geräts für zwei Lagerschächte von oben.
- **Fig. 5**: zeigt einen Schnitt entlang Linie A-A von Fig. 4.
- **Fig. 6**: zeigt einen Schnitt entlang Linie B-B von Fig. 4.

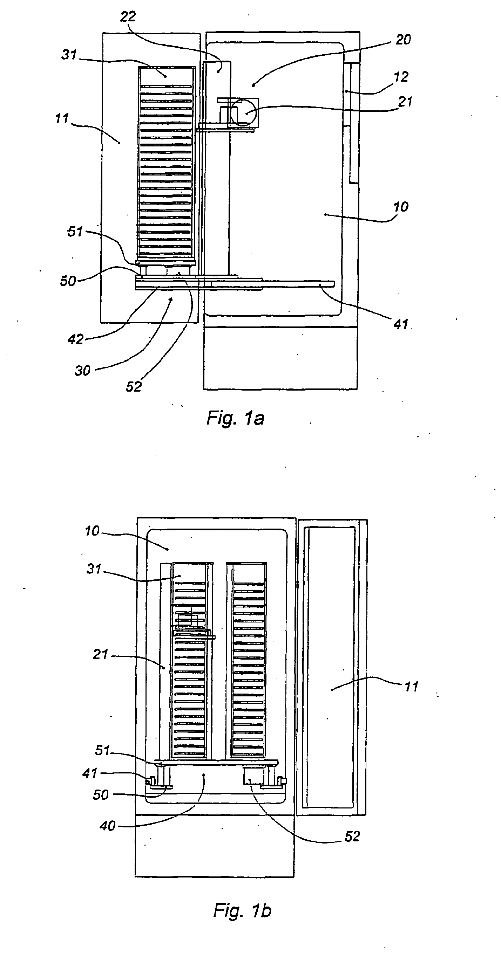

In der **Fig. 1** ist ein Klimaschrank mit einer Klimakammer **10** abgebildet. Im Innern der Klimakammer ist ein Lagersystem **30** angeordnet. Das Lagersystem besteht aus wenigstens einem herausnehmbaren Lagerschacht **31**, der übereinander angeordnete Lagerstellen zur Aufnahme von Lagerobjekten aufweist, und einer Transportvorrichtung **20**, die zum automatischen Transport der Lagerobjekte zwischen dem Lagerschacht und einem umgebenden System dient. Jeder der vorzugsweise mehreren Lagerschächte **31** besitzt eine Vielzahl von übereinander angeordneten Lagerstellen, auf welche die horizontal und vertikal verfahrbare und um eine vertikale Achse schwenkbare Transporteinrichtung 20 zugreifen kann. Eine Ausführung entsprechender Komponenten ist in CH 690 645 offenbart.

Auf der Rückseite des Klimaschrankes ist eine Hilfstüre **12** für das automatische Be- und Entladen des Lagergutes mittels der Transportvorrichtung **20** angeordnet. Auf die Lagerschächte kann manuell über eine Benutzertüre **11** zugegriffen werden.

Auf einer Grundplatte **50** ist neben einer Liftsäule **22** mit einem Transportlift **21** der Transportvorrichtung eine Trägerplatte **51** angeordnet. Die Grundplatte **50** ist vorzugsweise als U-förmig geschnittenes Blech ausgestaltet, mit Seitenschenkeln entlang den Seitenwänden des Klimaschranks und einer Basis parallel entlang der Rückwand des Klimaschranks. Die Lagerschächte **31** sind auf der Trägerplatte **51** angeordnet, d.h. die Trägerplatte 51 dient als Träger für die Lagerschächte 31. Die Trägerplatte **51** ist gegenüber der Grundplatte **50** horizontal mittels eines Verschiebemotors **52** bewegbar gelagert. Die Grundplatte **50** ist auf Verschiebewagen **42** auf Verschiebeschienen **41** gelagert, d.h. Verschiebewagen **42** und Verschiebeschienen **41** bilden ein Führungsmittel, in welchem die Grundplatte **50** geführt relativ zum Klimaschrank bewegt werden kann. Die Verschiebeschienen sind an den Seitenwänden der Klimakammer horizontal so befestigt, dass das Lagersystem durch die Benutzertüre aus der Klimakammer verschoben werden kann.
Die Trägerplatte ist so auf den Verschiebeschienen **41** gelagert, dass sie durch die Türe **11** aus dem Klimanutzraum **10** bewegt werden kann. Durch dieses Herausziehen des Transportsystems wird der Innenraum und insbesondere der Boden der Klimakammer zugänglich. Einstell- und Servicearbeiten am Transportsystem werden durch die verbesserte Zugänglichkeit vereinfacht. Mittels der Horizontalbewegung der Trägerplatte **51** zu der Grundplatte **50** lässt sich beispielsweise eine Schüttelbewegung der Lagerschächte implementieren.

Die **Fig. 1b** zeigt eine Frontansicht der Anordnung. Die Grundplatte **50** ist so ausgebildet, dass sie zusammen mit der Trägerlatte **51** eine Brücke **40** bildet. Hierzu ist die Trägerplatte **51** auf den seitlichen Schenkeln der tiefer gelegenen Grundplatte **50** mittels seitlichen Stützen befestigt, so dass sich ein frei zugänglicher Raum im Bereich der Brücke **40** bildet. Die Brücke verbessert die Zugänglichkeit zum Boden der Klimakammer ohne den Nachteil zusätzlicher Bauhöhe aufzuweisen.

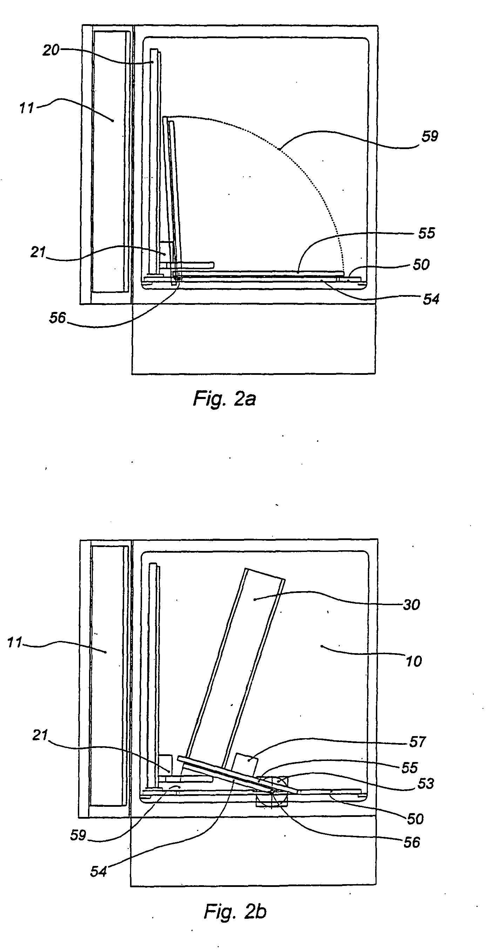

Die **Fig. 2a** zeigt ein Ausführungsbeispiel mit einer um eine Schwenkachse **56** in einem Schwenklager drehbar gelagerten Trägerplatte bzw. Schwenkplatte **54**. Die Schwenkachse **56** verläuft vorzugsweise horizontal und senkrecht zur vorderen Türöffnung des Klimaschranks. Sie bildet ein Führungsmittel bei der Schwenkbewegung der Schwenkplatte gegenüber dem Klimaraum. Auf der Schwenkplatte ist ein Drehteller **55** gelagert. Der Drehteller dient der Aufnahme der Lagerschächte. Die Schwenkplatte liegt in einer Ebene mit der Grundplatte und die Schwenkachse verläuft durch die Grundplatte. Beim Wegschwenken der Schwenkplatte um den Schwenkwinkel **59** bildet sich eine Öffnung in der Grundplatte, die eine Zugänglichkeit zum Boden der Klimakammer ermöglicht. Bei dieser Anordnung muss die Zugänglichkeit des Bodens der Klimakammer nicht über den Nachteil zusätzlicher Bauhöhe erkauft werden. Um die Schwenkplatte in die in Fig. 2a gezeigte, praktisch vertikale Position zu schwenken, müssen lediglich die Lagerschächte **31** aus dem Klimaschrank entfernt werden.

Bei dem Ausführungsbeispiel in der Fig. 2b wird die Schwenkbewegung der Schwenkplatte durch einen Schwenkmotor 53 ausgeführt. Ein Drehtellerantrieb 57 ist zusammen mit dem Drehteller 55 auf der Schwenkplatte 54 gelagert. Hierdurch kann bei aktivierter Drehbewegung des Drehtellers 57 in geschwenktem Zustand eine Rührbewegung in dem in den Lagerschächten gelagerten Lagergut realisiert werden.

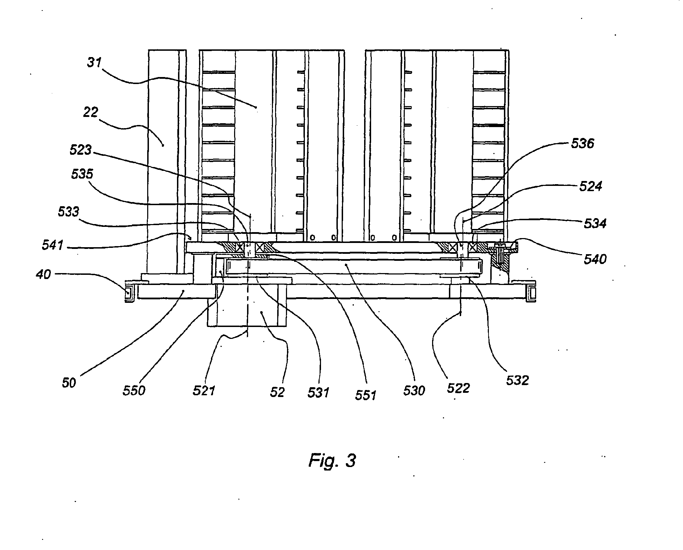

Die **Fig. 3** zeigt eine detaillierte Darstellung einer Anordnung mit beweglicher Trägerlatte **51** zur Verdeutlichung einer weiteren erfindungsgemässen Anordnung. In dieser Anordnung ist der Klimaschrank dazu ausgestaltet, die Lagerschächte bzw. das Lagergut zu rütteln.

Die Trägerplatte **51** ist mittels zweier Achsenlager **533, 534** einerseits und zweier Reiblager **540, 541** anderseits auf der stationären Grundplatte **50** gelagert. Jeweils ein Exenterlager ist exzentrisch in einem Riemenrad **531, 532** angeordnet und jeweils eine Achse **535, 536** verläuft durch den Innenring der Achsenlager **533, 534**. Das antriebsseitige Riemenrad **531** ist kraftschlüssig mit der Motorachse des Verschiebemotors **52** verbunden, während das andere Riemenrad **532** über einen Zahnriemen **530** mit der Antriebsseite synchron verbunden ist. Werden die Riemenräder **531, 532** in Rotation versetzt, wird die Trägerplatte 51 über die Achsen **535, 536** und die Achsenlager **533**, **534** in eine Vortexbewegung versetzt, d.h. gerüttelt. Zusätzliche Reiblager **540, 541**, die unzulässige Momente auf die Achsenlager verhindern, sind so ausgeführt, dass die Trägerplatte **51** der Bewegung folgen kann. Die Amplitude der Vortexbewegung entspricht dem doppelten Abstand der Riemenrad-Rotationsachse **521, 522** und der Exzenterlager-Rotationsachsen **523, 524**. Die Liftsäule **22** folgt dieser Bewegung nicht. Um die Lagerschächte bei einem Zugriff des Transportsystems stets in gleiche Position zur Liftsäule zu bringen, ist ein Positioniersensor **550** auf der Grundplatte **50** vorgesehen. Der Positioniersensor dient zur Positionsmessung bzw. Positionierung und steuert den Verschiebemotor **50** so, dass eine mit dem Riemenrad kraftschlüssig verbundene Schaltplatte **551** das Riemenrad stets unter gleichem Winkel anhält, so dass eine bekannte, d.h. definierte relative Position zwischen den Lagerschächten 31 und der Transportvorrichtung 20 bzw. dem Klimanutzraum beibehalten werden kann, so dass ein Zugriff der Transportvorrichtung 20 auf die Lagerschächte 31 gewährleistet ist.

Je nach Ausführung kann der Träger bzw. die Trägerplatte **51** gegenüber dem Klimanutzraum drehfest oder drehbar angeordnet sein. Insbesondere kann die Trägerplatte z.B. auch die Bodenplatte eines Karussells bilden oder tragen, wobei die Lagerschächte im Karussel angeordnet ist.

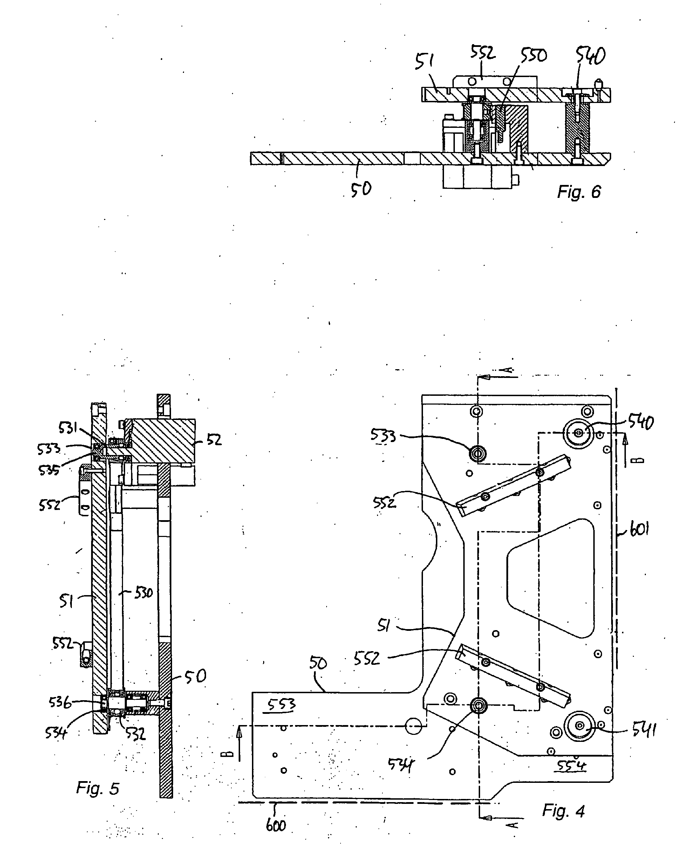

Fig. 4 bis 6 zeigen eine konkrete Ausführung von Grund- und Trägerplatte **50** bzw. **51** eines Geräts mit zwei Lagerschächten und Schüttelmechanismus. Dabei entsprechen die Teile jenen gemäss Fig. 3. Zusätzlich ersichtlich sind Befestigungsleisten **552** auf der Trägerplatte **51** zur Befestigung der Lagerschächte. Ebenfalls ersichtlich ist in dieser Ausführung eine im Wesentlichen L-förmige Gestaltung der Grundplatte **50**, die einen Schenkel **553** parallel zu einer Seitenwand 600 und eine Basis **554** parallel zur Vorderwand **601** des Nutzraums aufweist, wodurch der Zugriff auf den Boden der Kammer vereinfacht wird. Die Transportvorrichtung **20** ist auf dem Schenkel 553 montiert.

### Bezugszeichenliste

Klimakammer **10**
Benutzertüre **11**
Hilfstüre **12**
Transportvorrichtung **20**
Transportlift **21**
Liftsäule **22**
Lagersystem **30**
Lagerschacht **31**
Brücke **40**
Verschiebeschienen **41**
Verschiebewagen **42**
Grundplatte **50**
Trägerplatte **51**
Verschiebemotor **52**
Schwenkmotor **53**
Schwenkplatte **54**
Drehteller **55**
Schwenkachse **56**
Drehtellerantrieb **57**
Schwenkwinkel **59**
Zahnriemen **530**
Riemenrad **531, 532**
Riemenradrotationsachse **521, 522**
Exzenterlagerrotationsachsen **523, 524**
Achsenlager **533, 534**
Achse **535, 536**
Reiblager **540, 541**
Positioniersensor **550**
Schaltplatte **551**
Befestigungsleisten **552**
Schenkel **553**
Basis **554**
Wände des Nutzraums **600, 601**

## Patentansprüche

1. Klimaschrank mit einer Klimakammer (10) zur Aufnahme eines Lagersystems (30), mit einer automatischen Hilfstüre (12), mit mehreren Lagerschächten (31) und mit einer Transportvorrichtung (20) zum automatischen Transport von Lagerobjekten durch die Hilfstüre (12) zwischen den Lagerschächten (31) und einem umgebenden System, wobei der mindestens eine Lagerschacht (31) auf einer Trägerplatte (51) angeordnet ist, wobei jeder Lagerschacht (31) übereinander angeordnete Lagerstellen zur Aufnahme der Lagerobjekte aufweist und wobei die Trägerplatte (51) drehfest zur Klimakammer (10) angeordnet ist,
**dadurch gekennzeichnet , dass**
die Trägerplatte (51) relativ zur Klimakammer beweglich ist und dass der Klimaschrank einen Antrieb (52) zum Rütteln der Trägerplatte (51) aufweist.

2. Klimaschrank nach Anspruch 1, wobei Führungsmittel (41, 42; 56), insbesondere eine oder mehrere Schienen, vorgesehen sind, in welchen die Trägerplatte (51) relativ zur Klimakammer (10) geführt beweglich ist.

3. Klimaschrank nach Anspruch 2, wobei die Trägerplatte (51) auf den Führungsmitteln durch eine Türe (11) mindestens teilweise aus der Klimakammer (10) hinaus verschiebbar ist, und insbesondere wobei die Lagerschächte auf der Trägerplatte (51) mindestens teilweise aus der Klimakammer (10) hinaus verschiebbar sind.

4. Klimaschrank nach einem der vorangehenden Ansprüche, wobei die Trägerplatte (51) horizontal relativ zur Klimakammer(10) verschiebbar ist.

5. Klimaschrank nach einem der vorangehenden Ansprüche, mit einer Grundplatte (50), wobei die Trägerplatte (51) relativ zur Grundplatte (50) beweglich auf der Grundplatte (50) angeordnet ist.

6. Klimaschrank nach Anspruch 5, **dadurch gekennzeichnet, dass** die Grundplatte (50) U- oder L-förmig ist und mindestens einen Schenkel (553) entlang einer Seitenwand des Klimaschranks und eine Basis (554) parallel entlang einer weiteren Wand des Klimaschranks aufweist, und insbesondere dass die Transporteinrichtung auf dem Schenkel angeordnet ist.

7. Klimaschrank nach einem der Ansprüche 5 oder 6, wobei die Grundplatte (50) relativ zur Klimakammer (10) beweglich ist, und insbesondere dass sie relativ zur Klimakammer geführt verschiebbar ist.

8. Klimaschrank nach Anspruch 7, wobei Führungsmittel (40, 41) vorgesehen sind, auf denen die Grundplatte (50) durch eine Türe (11) des Klimaschranks mindestens teilweise aus der Klimakammer (10) hinaus bewegbar ist.

9. Klimaschrank nach einem der vorangehenden Ansprüche mit einer Positionsmessvorrichtung (550) für eine automatische Positionierung oder Positionserkennung der Trägerplatte (51) relativ zur Klimakammer (10) bzw. relativ zur Transporteinrichtung (20).

10. Klimaschrank nach einem der vorangehenden Ansprüche, aufweisend
einen Antrieb zum Bewegen der Trägerplatte (51) und
Mitteln (550) zum Anhalten des Motors in einer Position, in welcher die Lagerschächte (31) in vordefinierter Stellung zur Transportvorrichtung (20) stehen.

11. Klimaschrank nach einem der vorangehenden Ansprüche, wobei die Trägerplatte (51) auf seitlichen Stützen angebracht und an einer tiefer als die Trägerplatte (51) angeordneten Grundplatte (50) befestigt ist.

12. Klimaschrank nach einem der vorangehenden Ansprüche mit einer Grundplatte, auf welcher die Trägerplatte (51) und die Transporteinrichtung angeordnet sind.

## Claims

1. Climatic cabinet with a climatic chamber (10) for receiving a storage system (30) with an automatic auxiliary door (12), with a plurality of storage racks (31) and with a transport device (20) for an automatic transport of storage objects through the auxiliary door (12) between the storage racks (31) and an enclosing system, wherein the at least one storage rack (31) is arranged on a support plate (51), wherein each storage rack (31) has storage positions for receiving the storage objects and wherein the support plate (51) is arranged rotatably fixed with respect to the climatic chamber,
**characterized in that** the support plate (51) is movable with respect to the climatic chamber and **in that** the climatic cabinet has an actuator (52) for shaking the support plate (51).

2. Climatic cabinet according to claim 1,
wherein guide means (41, 42; 56), particularly one or more rails, are provided, in which the support plate (51) is movable in a guided way with respect to the climatic chamber (10).

3. Climatic cabinet according to claim 2,
wherein the support plate (51) is shiftable on the guide means through a door (11) at least partly out of the climatic chamber (10), and particularly wherein the storage racks are shiftable on the support plate (51) at least partly out of the climatic chamber (10).

4. Climatic cabinet according to one of the preceding claims, wherein the support plate (51) is shiftable horizontally with respect to the climatic chamber (10).

5. Climatic cabinet according to one of the preceding claims, with a base plate (50), wherein the support plate (51) is arranged on the base plate (50) in a movable way with respect to the base plate (50).

6. Climatic cabinet according to claim 5, **characterized in that** the base plate (50) has an U- or L-shape and has at least an arm (553) along a side wall of the climatic cabinet and a base (554) parallel along a further wall of the climatic cabinet, and particularly **in that** the transport device is arranged on the arm.

7. Climatic cabinet according to one of the claims 5 or 6, wherein the base plate (50) is movable with respect to the climatic chamber (10), and particularly is shiftable in a guided way with respect to the climatic chamber.

8. Climatic cabinet according to claim 7,
wherein guide means (40, 41) are provided, on which the base plate (50) is movable at least partly out of the climatic chamber (10) through a door (11) of the climatic cabinet.

9. Climatic cabinet according to one of the preceding claims with a position measurement device (550) for an automatic positioning or position detection of the support plate (51) with respect to the climatic chamber (10) or with respect to the transport device (20) respectively.

10. Climatic cabinet according to one of the preceding claims, having
an actuator for moving the support plate (51)
and
means (550) for stopping the motor in a position in which the storage racks (31) are located in a predefined position with respect to the transport device (20).

11. Climatic cabinet according to one of the preceding claims, wherein the support plate (51) is fitted onto side bearings and attached to a base plate (50) which is arranged lower than the support plate (51).

12. Climatic cabinet according to one of the preceding claims with a base plate on which the support plate (51) and the transport device are arranged.

## Revendications

1. Armoire climatique avec un compartiment climatique (10) pour recevoir un système de stockage (30), avec une porte auxiliaire (12) automatique, avec une pluralité de récipients de stockage (31) et avec un dispositif de transport (20) pour le transport automatique d'objets à stocker par la porte auxiliaire (12) entre les récipients de stockage (31) et un système entourant, l'au moins un récipient de stockage (31) étant arrangé sur une plaque de support (51), chaque récipient de stockage (31) ayant des positions de stockage pour la réception des objets de stockage et la plaque de support (51) étant arrangée de manière fixe et rotative par rapport au compartiment climatique (10),
**caractérisée en ce que** la plaque de support (51) est mobile par rapport au compartiment climatique et **en ce que** l'armoire climatique a un entraînement (52) pour vibrer la plaque de support (51).

2. Armoire climatique selon la revendication 1, des moyens de guidage (41, 42; 56), particulièrement une ou plusieurs barres, étant prévus, dans lesquels la plaque de support (51) est guidée de manière mobile par rapport au compartiment climatique (10).

3. Armoire climatique selon la revendication 2, la plaque de support (51) étant déplaçable sur les moyens de guidage par une porte (11) au moins partiellement hors du compartiment climatique (10), et particulièrement les récipients de stockage étant déplaçable sur la plaque de support (51) au moins partiellement hors du compartiment climatique (10).

4. Armoire climatique selon l'une des revendications précédentes, la plaque de support (51) étant déplaçable horizontalement par rapport au compartiment climatique (10).

5. Armoire climatique selon l'une des revendications précédentes, avec une plaque de base (50), la plaque de support (51) étant arrangée sur la plaque de base (50) de manière mobile par rapport à la plaque de base (50).

6. Armoire climatique selon la revendication 5, **caractérisée en ce que** la plaque de base (50) est en forme de U ou L et elle au moins une branche (553) le long d'une paroi latérale de l'armoire climatique et une base (554) parallèle le long d'encore une paroi de l'armoire climatique, et particulièrement **en ce que** le dispositif de transport est arrangé sur la branche.

7. Armoire climatique selon l'une des revendications 5 ou 6, la plaque de base (50) étant mobile par rapport au compartiment climatique (10), et particulièrement étant déplaçable de manière mobile par rapport au compartiment climatique.

8. Armoire climatique selon la revendication 7, des moyens de guidage (40, 41) étant prévus, sur lesquels la plaque de base (50) est déplaçable par une porte (11) au moins partiellement hors du compartiment climatique (10).

9. Armoire climatique selon l'une des revendications précédentes avec un dispositif de mesure de position (550) pour un positionnement ou une détection de position automatique de la plaque de support (51) par rapport au compartiment climatique (10) ou bien par rapport au dispositif de transport (20).

10. Armoire climatique selon l'une des revendications précédentes, ayant
un entraînement pour entraîner la plaque de support (51) et
des moyens (550) pour arrêter le moteur dans une position dans laquelle les récipients de stockage (31) sont positionnés dans une position prédéfinie par rapport au dispositif de transport (20).

11. Armoire climatique selon l'une des revendications précédentes, la plaque de support (51) étant fixée sur des butes latérales et attachée à une plaque de base (50) arrangée plus bas que la plaque de support (51).

12. Armoire climatique selon l'une des revendications précédentes avec une plaque de base, sur laquelle la plaque de support (51) et le dispositif de transport sont arrangés.
